Europäisches Patentamt

European Patent Office

Office européen des brevets

(11) Veröffentlichungsnummer : **0 075 184**
**B1**

# (12) EUROPÄISCHE PATENTSCHRIFT

(45) Veröffentlichungstag der Patentschrift :
30.01.85

(21) Anmeldenummer : 82108237.7

(22) Anmeldetag : 08.09.82

(51) Int. Cl.⁴ : **G 01 N 33/48, G 01 N 33/52,
G 01 N 31/22, B 01 L 3/02**

(54) Testsystem und Verfahren zur Bestimmung von Inhaltsstoffen von Flüssigkeiten.

(30) Priorität : 17.09.81 DE 3137014

(43) Veröffentlichungstag der Anmeldung :
30.03.83 Patentblatt 83/13

(45) Bekanntmachung des Hinweises auf die Patenterteilung : 30.01.85 Patentblatt 85/05

(84) Benannte Vertragsstaaten :
BE CH DE FR GB IT LI NL SE

(56) Entgegenhaltungen :
DE-B- 2 557 060
US-A- 3 917 453

(73) Patentinhaber : Merck Patent Gesellschaft mit beschränkter Haftung
Frankfurter Strasse 250
D-6100 Darmstadt (DE)

(72) Erfinder : Helger, Roland, Dr.
Ludwigshöhstrasse 85
D-6100 Darmstadt (DE)
Erfinder : Limbach, Berthold, Dr.
Otto-Hahn-Strasse 8
D-6104 Seeheim-Jugenheim 4 (DE)

**0 075 184**

**Beschreibung**

Die Erfindung betrifft ein Testsystem und Verfahren zur Bestimmung von Inhaltsstoffen von Flüssigkeiten, wobei das Testsystem im wesentlichen aus mindestens einer, in einem Reaktionsgefäß angeordneten preßfähigen Matrix besteht.

Die Bestimmung von Inhaltsstoffen komplexer Systeme, insbesondere von biologischen Flüssigkeiten, erfordert eine genau festgelegte Versuchsdurchführung, wobei die Probe mit der zu bestimmenden Substanz verschiedene Reaktionsprozesse durchläuft. Dabei sind je nach der verwendeten Methode und der Anzahl der Proben Zugabe und Zeitpunkt der zu mischenden Reagenzien verschieden. Ein nach Ablauf der Reaktion auszuwertendes Meßsignal, das für die Methode und die zu bestimmende Substanz charakteristisch ist, führt schließlich zur Konzentrationsermittlung der gesuchten Substanz. Entsprechend der Komplexität solcher Prozesse sind bisher eine Reihe unterschiedlicher Verfahren erprobt worden, die ein Optimum an Zuverlässigkeit, Einfachheit und Wirtschaftlichkeit erbringen sollen. Vor allem in der klinischen Chemie wurden als Folge der zum Teil sehr hohen Analysenzahlen Methoden entwickelt, die die Durchführung vieler Bestimmungen auf möglichst einfache Weise und in kurzer Zeit ermöglichen. Einerseits wird dies durch den Einsatz mechanisierter Analysensysteme, andererseits durch die Bereitstellung der notwendigen Reagenzien und Reaktionsgemische in weitgehend vorbereiteter Form erreicht. Dabei kommen vor allem Analysensysteme zum Einsatz, bei denen die Probe mit Reaktionslösungen aus Vorratsbehältern manuell oder voll mechanisiert gemischt wird. Die Bereitstellung der Lösungen, die eventuell erforderliche Vorbereitung der Probe sowie die Überwachung und Reinigung des Verteilungs- und Mischungssystems bedingen jedoch eine Vielzahl von Arbeitsschritten und möglichen Fehlerquellen. Insbesondere die relativ geringe Haltbarkeit einiger Analysensubstanzen in Lösung erfordert sehr oft einen Austausch nicht aufgebrauchter Lösungen. Zudem verlangen die Testbedingungen stets eine exakt reproduzierbare Zugabe verschiedener Volumina unterschiedlicher Lösungen, was bei automatisierten Systemen ein einfaches Auswechseln von Analysenverfahren unmöglich macht.

Die beschriebenen Schwierigkeiten führten zur Entwicklung von verschiedenen Testsystemen. So ist zum Beispiel bekannt, die Reagenzien in fester Form entweder als imprägnierte Trägermaterialien, wie getränkte Filterpapiere, oder als Lyophilisate in entsprechenden Reaktionsgefäßen mit der zu untersuchenden Probe in Kontakt zu bringen ; bei derartigen Systemen ist nur noch ein Zupipettieren von Lösungsmittel und Untersuchungsprobe erforderlich. Ein Anbringen verschiedener lyophilisierter Reagenzien an unterschiedlichen Stellen erlaubt dabei die Durchführung hintereinandergeschalteter Reaktionen. Diese Systeme stellen für die Testdurchführung eine Vereinfachung dar, bieten aber bei der Herstellung der entsprechenden Reaktionsgefäße sehr große Probleme. Vor allem bleibt diesen Ausführungsformen der für alle wäßrigen Systeme gemeinsame Nachteil, keine für die Analyse von Körperflüssigkeiten erforderlichen Auf- und Abtrennungen damit durchführen zu können und alle im Verlauf einer Analyse zugegebenen Reagenzien mitführen zu müssen.

Bei den sogenannten trockenen Analysenverfahren entfällt dagegen von vornherein ein Teil dieser Probleme. Hierbei wird die zu untersuchende Probe auf einen meist saugfähigen Träger aufgebracht, der im einfachsten Fall in einer einzigen Matrixschicht alle notwendigen Reagenzien in optimaler Konzentration enthält ; dadurch entfallen alle sonst üblichen Pipettierschritte. Außerdem stellt die Lagerung solcher trockenen Systeme in bezug auf die Haltbarkeit kein schwerwiegendes Problem dar. Als Nachteil dieser einstufigen Systeme erweist sich jedoch, wie auch bei den wäßrigen Verfahren, die fehlende Vor- und Aufbereitungsmöglichkeit sowie die mangelnde Möglichkeit, störende Substanzen auszuschalten.

Aber auch bei Testsystemen mit mehrschichtig angeordneten, trockenen Materialien, die zum Beispiel Schichten für die Abtrennung von Störsubstanzen, Konzentrierungszonen sowie verschiedene Reaktions- und Sperrbereiche enthalten können, ist die Aufnahme und der Weitertransport der zu untersuchenden Flüssigkeit sowie der Ablauf der einzelnen Reaktionsschritte allein von der Diffusion und Kapillarwirkung abhängig und dadurch praktisch weder kontrollierbar noch steuerbar. So ist es nicht möglich, die Reaktion an einer bestimmten Stelle zu stoppen oder die Verweildauer in einer Reaktionszone zu beeinflussen.

Aus dem US-Patent 3,917,453 ist eine Testvorrichtung bekannt, die ein absorbierendes Material zur Aufnahme der Probelösung und eine ebenfalls aus einem absorbierenden Material bestehende preßfähige Schicht enthält, die der Dosierung und dem Transport der zu untersuchenden Flüssigkeit dient. Diese Schicht ist zwischen zwei Abdeckschichten angeordnet, die die notwendigen Reagenzien enthalten und mit der zu bestimmenden Substanz unter Bildung eines Farbstoffs reagieren kann, der kolorimetrisch ausgewertet wird. Diese Vorrichtung ist jedoch mit einer Reihe von Nachteilen behaftet : Quantitative Bestimmungen, eine photometrische Auswertung und eine Automatisierung sind nicht möglich ; die Durchführung von Reaktionen, die mehrere hintereinander ablaufende Reaktionsstufen erfordern, und eventuell erforderliche Abtrennungen unerwünschter Substanzen zwischen zwei Reaktionszonen ist mit dieser Vorrichtung ebenfalls nicht möglich.

Der vorliegenden Erfindung liegt die Aufgabe zugrunde, Vorrichtungen und Verfahren zur Bestimmung von Inhaltsstoffen von Körperflüssigkeiten zur Verfügung zu stellen, mit denen die geschilderten Nachteile vermieden werden können.

2

Erfindungsgemäß wurde diese Aufgabe durch ein Testsystem gelöst, das aus einem Reaktionsgefäß besteht, das mindestens eine preßfähige Matrix enthält, womit ein einfacher, von der Diffusion unabhängiger Flüssigkeitstransport durchgeführt werden kann, der sowohl manuell als auch automatisch steuerbar ist.

Gegenstand der Erfindung ist ein Testsystem zur Bestimmung von Inhaltsstoffen von Flüssigkeiten unter Verwendung preßfähiger, mit Reagenzien imprägnierter Matrices, dadurch gekennzeichnet, daß es mindestens eine imprägnierte, preßfähige Matrix in einem vorzugsweise zylindrischen Reaktionsgefäß und eine Vorrichtung enthält, durch die die Matrix nach Aufnahme der zu untersuchenden Flüssigkeit unter Abgabe des Reaktionsproduktes komprimiert wird.

Ferner betrifft die Erfindung ein Verfahren zur Bestimmung von Inhaltsstoffen von Flüssigkeiten unter Verwendung preßfähiger, mit Reagenzien imprägnierter Matrices, die dadurch gekennzeichnet sind, daß man die zu untersuchende Flüssigkeit auf eine in einem vorzugsweise zylindrischen Reaktionsgefäß angeordnete Matrix aufbringt und das Reaktionsprodukt durch Komprimieren der Matrix aus dieser entfernt und entweder direkt oder erst nach einer oder mehreren weiteren Passagen durch imprägnierte Matrices einer Auswertung zuführt.

Überraschenderweise hat sich gezeigt, daß das erfindungsgemäße Testsystem die Anwendung und Wirksamkeit der Analysenmethoden wie auch der Reagenzien in gleicher Weise ermöglicht wie ein vergleichbares System in wäßriger Lösung, darüber hinaus jedoch zusätzliche Verfahren der Probenvorbereitung, wie Zentrifugationen, Filtrationen oder chromatographische Trennungen, in sich vereinigt oder sie ersetzt.

Das Testsystem für die Analyse von Flüssigkeiten kann in einzelne Bereiche aufgegliedert werden, in denen voneinander getrennt alle für die Analyse notwendigen Trennungs-, Reaktions- und Bestimmungsreaktionen ablaufen. Dies schließt zum Beispiel bei der Analyse von Körperflüssigkeiten neben dem für die eigentliche Bestimmung einer Substanz erforderlichen Reaktionsbereich und ggf. einen Anzeigebereich auch Vorbereitungsbereiche, etwa zur Enteiweißung oder Abtrennung von Störsubstanzen, ein. Dabei sind Art, Anzahl und Anordnung der einzelnen Bereiche frei kombinierbar. So können zum Beispiel bei speziellen Bestimmungen gesonderte Vorbereitungsbereiche entfallen, oder es können an einen gemeinsamen Vorbereitungsbereich mehrere verschiedene Reaktionsbereiche gekoppelt werden.

Bei dem erfindungsgemäßen Testsystem sind alle zur Reaktion erforderlichen Reagenzien von Anfang an gleichmäßig in der gesamten Matrix lyophilisiert, immobilisiert oder in anderer Weise eingeschlossen enthalten. Ein Auftragen der zu untersuchenden Flüssigkeit führt zu einer direkten Durchmischung der Reaktionspartner über den gesamten Matrixbereich. Die einzelnen Reaktionsbereiche sind innerhalb des Reaktionssystems klar getrennt, das heißt, es erfolgt keine vorzeitige Diffusion oder Vermischung. Darüber hinaus bietet die erfindungsgemäße Vorrichtung eine Reihe weiterer Vorteile gegenüber dem Stand der Technik. So kann zum Beispiel eine Verbesserung der Durchmischung und eine Erhöhung der Reaktionsgeschwindigkeit durch zusätzliche mechanische Manipulationen, wie Komprimieren der Matrixschicht während der Reaktion, erreicht werden ; das Testsystem erlaubt die Verwendung verschiedener oder unterschiedlich vorbereiteter Matrices für die einzelnen Testbereiche ; die Auswertung ist nicht nur visuell, sondern auch photometrisch möglich ; die Durchführung kann manuell und mechanisiert erfolgen ; die Verwendung der Matrix ermöglicht eine genaue Dosierung und leichte Handhabung von Substanzen und Flüssigkeiten im Testsystem.

Die erfindungsgemäße Vorrichtung besitzt damit die bei den beschriebenen mehrschichtigen trockenen Verfahren erwähnten Vorteile (keine instabilen Reagenzlösungen, keine Pipettierschritte, mehrere Testbereiche), doch ist darüber hinaus der Gesamtablauf der Reaktion nicht diffusionsabhängig und daher, wie bei den entsprechenden wäßrigen Systemen, beeinflußbar und beliebig unterbrechbar. Ein mögliches Baukastenprinzip erlaubt ein leichtes Austauschen von Teilbereichen und Gesamttestsystemen.

Grundlage des Testsystems ist eine Matrix aus einem flexiblen, elastischen, saugfähigen Material, die als Träger für Reagenz und Probe sowie als Reaktionsraum dient, als Trennmedium fungiert und es gestattet, Lösungen möglichst vollständig, jedenfalls aber reproduzierbar, reversibel freizugeben. Diese Matrix ist in der Lage, ein bestimmtes Volumen einer Probeflüssigkeit vollständig und gleichmäßig verteilt in sich aufzunehmen und zu speichern, um es bei einem angelegten Druck möglichst vollständig abzugeben ; dieser Vorgang ist beliebig wiederholbar. Die Matrix muß darüber hinaus eine genügende mechanische und chemische Stabilität besitzen. Unter flexibel und elastisch soll in diesem Zusammenhang eine bei gleichbleibenden äußeren Bedingungen konstante Raum- und Formausfüllung der Matrix verstanden werden, ohne daß hierzu ein Stützgefäß erforderlich ist ; sie soll durch Anwendung von Druck reversibel verändert werden können. Die Matrix kann aus einem Stück, aus mehreren Segmenten oder aus einer Vielzahl freier oder nachträglich miteinander, zum Beispiel durch Verkleben oder Vernetzen, verbundener Teile bestehen, die alle oder zum überwiegenden Teil den entsprechenden Anforderungen genügen.

Geeignete Matrixmaterialien sind natürliche und synthetische schwamm- und schaumartige, poröse Stoffe, zum Beispiel offenzellige Schaumstoffe und Verbundschaumstoffe verschiedenen Ursprungs sowie entsprechend elastisch gemachte Mikrokapseln, Granulate oder andere Formkörper aus saugfähigen Materialien. Vorzugsweise zählen hierzu offenzellige Polyurethanschäume, insbesondere Polyester- und Kaltschäume.

Darüber hinaus sind aber auch spezielle Schaumstoffe als Matrix verwendbar, wie SH-Gruppen enthaltende Schaumstoffe bei der Verwendung von Enzymen, die SH-Gruppen benötigen, oder auch transparente Polymere für visuelle Auswertungen.

Die Stauchhärte dieser Materialien sollte im Bereich von etwa 2 bis 10 kPa liegen, wobei solche mit einer Stauchhärte unter 2 kPa schlechte mechanische Eigenschaften zeigen, solche über 10 kPa erfordern einen zu großen Druck bei der Flüssigkeitsabgabe. Das Raumgewicht hat keinen wesentlichen Einfluß auf das verwendbare Material.

Die im Handel erhältlichen Schaumstoffe weisen zum Teil eine starke Enzymhemmung auf ; bei einigen kann es während der Durchführung des erfindungsgemäßen Verfahrens zu starken Trübungen kommen. Die Hemmungen lassen sich jedoch in der Regel .vollständig durch den Zusatz von Komplexbildnern eliminieren. Darüber hinaus können weitere Verbesserungen durch entsprechende Vorbehandlung erhalten werden. Für die kinetische Bestimmung von Enzymen in Flüssigkeiten können sich dagegen enzymhemmende Matrices als besonders vorteilhaft erweisen. So ist es möglich, die Matrix, die die erforderlichen Reagenzien enthält, mit der zu untersuchenden Flüssigkeit zu tränken, wobei die eigentliche Bestimmungsreaktion nicht oder nur langsam abläuft. Beim Überführen der Gesamtlösung aus der Matrix in den Testbereich bleiben die unerwünschten Hemmstoffe zurück, und die kinetische Bestimmung ist bei relativ geringer Anfangsextinktion möglich.

Das Einbringen der Reagenzien in die Matrix erfolgt in der Regel durch Lyophilisieren der mit Reagenzlösung getränkten Schaumstoffsegmente. Dies hat neben einer größeren Stabilität der aufgetragenen Substanzen auch den Vorteil, daß nur eine geringe Menge an Reagenzien erforderlich ist. So wurde überraschenderweise gefunden, daß etwa bei einer Abtrennung von Protein aus einer Untersuchungsprobe mit Hilfe eines Koagulierungsmittels, wie Trichloressigsäure, eine bis zu zehnfach geringere Menge an Koagulierungsmittel erforderlich ist als bei den üblichen Fällungsmethoden. Damit wird durch Vermeidung der sonst durch die notwendige Neutralisation bedingten hohen Salzbeladung ein Hintereinanderschalten von verschiedenen Testbereichen nach der Erfindung begünstigt.

Im allgemeinen werden alle für die Funktion des betreffenden Bereiches erforderlichen Reagenzien, getrennt für jeden Bereich, auf die Matrix aufgegeben. In speziellen Fällen, zum Beispiel bei Unverträglichkeit von Reagenzien oder gleichzeitigem Ablauf mehrerer Reaktionen mit verschiedenen Reagenzien, kann es auch zweckmäßig sein, die erforderlichen Reagenzien getrennt voneinander aufzugeben. Dies geschieht vorzugsweise durch Zusammensetzen der Gesamtmatrix in diesem Bereich aus einzelnen Matrixsegmenten, die jeweils verschiedene Substanzen enthalten. Die Matrixsegmente können in Form und Größe beliebig gestaltet sein, sie können zum Beispiel aus Kreissegmenten, Scheiben oder Kugeln bestehen, die voneinander unabhängig sind, oder aber vor bzw. nach Einbringen in das Reaktionsgefäß miteinander durch geeignete Mittel wie Verleimen oder mechanische Befestigung verbunden werden.

In bestimmten Fällen kann es erforderlich sein, daß zwar eine vollständige Übertragung der Probelösung von einem Bereich zum nächsten gewährleistet sein muß, aber die im nächsten Bereich störenden Reagenzien nicht mitübertragen werden sollen. Dies erfordert die Zwischenschaltung einer entsprechend präparierten Matrix bzw. eine Immobilisierung der Reagenzien, die zum Beispiel adsorptiv, durch Einkapselung oder durch chemische Methoden erfolgen kann. Auf diese Weise ist es möglich, mehrere hintereinandergeschaltete Bereiche mit sehr unterschiedlichen Reaktionsbedingungen, zum Beispiel unterschiedlichen pH-Werten oder mit sich gegenseitig störenden Reagenzien, zu koppeln.

Die verschiedensten Möglichkeiten der Immobilisierung von Stoffen an polymere Trägermaterialien durch chemische Methoden sind aus der Literatur bekannt. Erwähnt seien zum Beispiel die Bindung an Träger mit funktionellen Gruppen oder die Vernetzung mit Glutardialdehyd oder Acrylamid. Auch Immobilisierungen von niedermolekularen Substanzen an Polyurethanschäumen, insbesondere an sogenannten Kaltschäumen, sind durch eine entsprechend adaptierte Variation des sogenannten Triazin-Verfahrens möglich (Biochem. J. 109, 137 (1968)). Dieses Verfahren gestattet es, eine Vielzahl von Verbindungen mit OH-, SH- oder $NH_2$-Gruppen an die Schaumstoffe mit einer Bindungsausbeute von ca. 1 $\mu$mol/ml Schaumstoff zu binden. Neben den Reagenzien selbst ist es selbstverständlich auch möglich, kurz- und langkettige, zum Beispiel an Polymere wie Stärke oder Dextrane gebundene Derivate zu binden oder in dem Schaumstoff physikalisch oder durch Einschluß zurückzuhalten.

Das Reaktionsgefäß hat zweckmäßigerweise die Form eines Zylinders mit konischem Auslauf ; vorzugsweise werden Pipetten, Injektionsspritzen oder sogenannte Einwegspritzen mit einem Durchmesser von 5 bis 30 mm und einer Länge von 10 bis 150 mm verwendet. Es können aber auch küvettenartige Reaktionsgefäbe verwendet werden. Diese Reaktionsgefäße können aus durchsichtigem oder zum Teil durchsichtigem Kunststoff oder festen Materialien wie Glas oder Metall bestehen. Bevorzugt sind flexible durchsichtige Kunststoffe.

Nach einer besonderen Ausführungsform enthält das Testsystem nicht nur eine Matrix, sondern mehrere übereinander angeordnete Matrices, die durch flüssigkeitsdurchlässige Anordnungen wie Stege, durchlöcherte Zwischenscheiben oder Verjüngungen des Reaktionsgefäßes voneinander getrennt sind. Durch die Form dieser Anordnungen und die Größe der Löcher in den Zwischenscheiben lassen sich die Fließ- und Diffusionsprozesse bei der Überführung der zu analysierenden Flüssigkeit zwischen den einzelnen Matrixbereichen beeinflussen. Die Zwischenscheiben können so in das Reaktionsgefäß eingepaßt werden, daß sie bei Anwendung eines bestimmten Druckes verschiebbar sind.

Falls das Reaktionsgefäß zwischen den einzelnen Matrices Verjüngungen besitzt, können diese durch seitlich von außen angelegten Druck verschlossen werden; wird dabei die Matrix ebenfalls zusammengepreßt, so tritt die Flüssigkeit aus dem Schaumstoff aus, um beim Entspannen wieder vollständig vom Schaumstoff aufgenommen zu werden. Der Preßvorgang ist auch durch Herabdrücken eines durchlöcherten Stempels durchführbar.

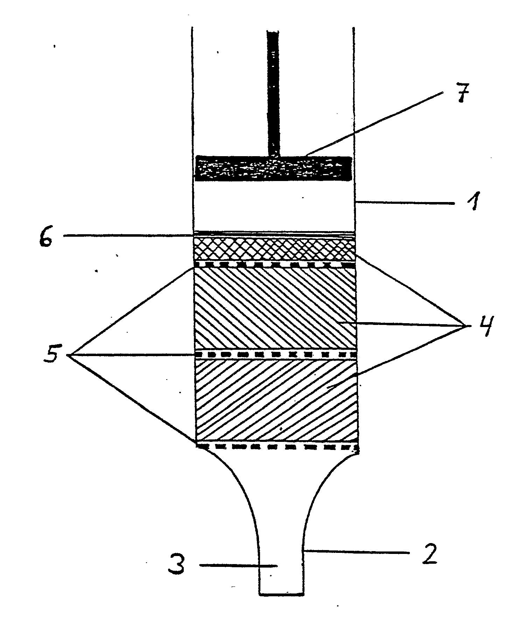

Die Abbildung zeigt eine schematische Darstellung einer erfindungsgemäßen Vorrichtung. Die dargestellte Ausführungsform besteht aus dem Reaktionsgefäß 1, das in einer Verengung 2 ausläuft, die als Küvette gestaltet ist. 3 ist entweder der Anzeigebereich des Testsystems oder Sammelbehälter für die außerhalb des Reaktionsgefäßes auszuwertende Flüssigkeit. Die mit Reagenzien imprägnierten Matrices 4 sind durch Zwischenscheiben 5 voneinander getrennt; die obere Schicht 6 stellt eine Verteilungsschicht dar, mit 7 ist der Stempel bezeichnet.

Die Durchführung des Verfahrens mit Hilfe der erfindungsgemäßen Vorrichtung erfolgt in der Weise, daß die zu untersuchende Probe durch ein übliches Auftragungssystem auf die obere Matrix des Reaktionsgefäßes aufgebracht wird; dabei wird auch das zur Verdünnung notwendige Lösungsmittel zugegeben. Es ist natürlich auch möglich, durch einfachen Kontakt mit der Probeflüssigkeit diese durch Kapillarwirkung in den Bereich dieser Matrixschicht zu saugen. Dieser Vorgang kann durch geeignete Konstruktion des oberen Teils der Reaktionsgefäßes, zum Beispiel durch Ausbildung einer ein bestimmtes Volumen fassenden Kammer, erleichtert werden. Auch der Einbau einer zusätzlichen Verteilungsschicht über der ersten Matrixschicht, die zum Beispiel aus einer Filterpapierschicht bestehen kann, ist möglich. Dabei kann die zu untersuchende Lösung vor oder nach der Auftragung verdünnt werden. Das Verfahren kann anschießend entweder sofort weiter durchgeführt werden oder das Reaktionsgefäß kann zwischengelagert werden.

Nach einer festgelegten Reaktionszeit wird die erste Matrixschicht durch eine dazu geeignete Vorrichtung, zum Beispiel einen Stempel, zusammengedrückt. Der Druck wird dabei so eingestellt, daß die Matrix zusammengepreßt wird, die Zwischenscheibe ihre Lage jedoch beibehält. Dadurch wird gewährleistet, daß die in der Matrix enthaltene Flüssigkeit auf die zweite Matrix übertragen wird. Die Eigenschaften der beschriebenen Matrix machen dabei eine Dichtung zwischen den einzelnen Matrices überflüssig.

Verschiedene Teilreaktionen können auf diese Weise getrennt voneinander durchgeführt werden, wie Enteiweißung, Auftrennung der Bestandteile des Vollblutes, Auftrennung von Isoenzymen, Immunreaktionen, spezifische Fällungen oder Komplexbildungen, enzymatische oder chemische Bestimmungsreaktionen. Darüber hinaus ist es möglich, Substanzen zusätzlich durch entsprechende Zwischenschichten zwischen zwei Bereichen zurückzuhalten. Diese Zwischenschichten können ebenfalls aus Schaumstoff, vorbehandeltem Schaumstoff oder auch aus Filterschichten aus Papier oder anderem saugfähigen Material bestehen.

Es ist wesentlich, daß die zu untersuchende Flüssigkeit reproduzierbar von einem Bereich zum anderen transportiert wird. Bevorzugt wäre eine quantitative Überführung, was jedoch aufgrund der verwendeten Matrixmaterialien nicht erreicht werden kann. Es werden normalerweise etwa 2 bis 15 % der Flüssigkeit von der Matrix zurückgehalten. Dieser Prozentsatz ist jedoch für das verwendete Matrixmaterial reproduzierbar und geht als konstanter Faktor in die Auswertung ein.

Die Auswertung erfolgt in an sich üblicher Weise nach allen gängigen Methoden. Bevorzugt sind optische oder reflektometrische Auswertungen.

Nach Passage der (letzten) Matrixschicht gelangt die Reaktionslösung entweder in eine Meßzelle innerhalb des Reaktionsgefäßes und wird innerhalb des Reaktionsgefäßes ausgewertet. Es ist aber auch möglich, die Reaktionslösung in eine außerhalb des Reaktionsgefäßes befindliche Meßzelle zu transportieren und die Auswertung darin vorzunehmen. Es sind aber auch spezielle Anzeigereaktionen durchführbar, wie z. B. eine Anzeige über Gasdiffusion oder eine elektrochemische Bestimmung.

Als zu untersuchende Flüssigkeiten wurden hauptsächlich Körperflüssigkeiten eingesetzt, da das erfindungsgemäbe Verfahren und Testsystem insbesondere zur Bestimmung der Inhaltsstoffe von Blut, Serum oder Harn geeignet ist.

Die Erfindung wird durch die nachfolgenden Beispiele näher erläutert:

Beispiel 1

Enteiweißung einer Serumprobe

Aus einem Polyurethan-Kaltschaum (Schaumstoff R 6590 der Firma Metzeler, Memmingen) wurden scheibenförmige Segmente (Ø 15 mm, Höhe 4 mm) ausgebohrt. Diese wurden mit 50 µl einer Trichloressigsäurelösung (2 mol/l) durch Auftragen in die Mitte des Segmentes getränkt und solange getrocknet, bis sie einen Trichloressigsäuregehalt von $5 \times 10^{-5}$ mol aufwiesen. Je ein derartig vorbereitetes Matrixsegment wurde in eine 10-ml-Einwegspritze eingeführt. Das Zentrum des Segmentes wurde jeweils mit 10 µl einer Probe von Seren (5,5 mg/dl Proteingehalt) versetzt, die verschiedene, definierte Glucosemengen enthielten. Anschließend wurde ein am Stempel der Spritze befestigtes Matrixsegment aus einem Polyurethan-Polyesterschaum (Schaumstoff ME 6060 der Firma Metzeler, Ø 15 mm, Höhe 16 mm), das mit 1,1 ml Wasser getränkt war, jeweils von oben zusammen mit dem Stempel eingeführt und bis zum Anschlag durchgedrückt. In der ausgedrückten Gesamtlösung wurde der Proteingehalt (Biuret-Verfahren) sowie der Glucosegehalt (Glucose-Dehydrogenase-Verfahren) nach folgender Formel bestimmt :

$$C = F \cdot E$$

wobei E die bei 365 nm gemessene Extinktion ist. Der Faktor F berücksichtigt Verdünnung, Blindwert und Glucoseverlust in der Matrix und wurde über Versuchsreihen mit Standardlösungen als 710 ermittelt.

| Glucose [mg/100 ml] | | Protein [g/100 ml] | |
|---|---|---|---|
| eingesetzt | gefunden | eingesetzt | gefunden |
| 20 | 19 | 5,5 | 0,1 |
| 34 | 32 | 5,5 | 0 |
| 50 | 52 | 5,5 | 0,2 |
| 70 | 74 | 5,5 | 0,1 |
| 100 | 97 | 5,5 | 0,1 |
| 141 | 135 | 5,5 | 0 |
| 167 | 174 | 5,5 | 0,1 |
| 250 | 245 | 5,5 | 0,1 |
| 500 | 478 | 5,5 | 0,2 |

Die Zahlenwerte zeigen, daß die Enteiweißung gelungen ist und die Glucosewerte die eingesetzten Konzentrationen mit zufriedenstellender Genauigkeit wiedergeben.

Beispiel 2

Glucosebestimmung

Aus einem Polyurethan-Polyesterschaum (Schaumstoff ME 6060 der Firma Metzeler, Memmingen) wurden zylindrische Matrixsegmente (Ø 15 mm, Höhe 16 mm) ausgebohrt und ausgewaschen. Die getrockneten Segmente wurden mit 1,1 ml einer Lösung getränkt, die

0,2 mol/l Phosphatpuffer, pH 7,6,
0,15 mol/l Natriumchlorid,
5 kU/l Glucose-Dehydrogenase,
0,11 kU/l Mutarotase,
1 mmol/l NAD⁺ (Nicotinamid-adenin-dinucleotid) und
25 mmol/l Ethylendiamintetraacetat

enthielt, und anschließend lyophilisiert. Die so hergestellten Reaktionsmatrices wurden in einer 10-ml-Einwegspritze durch Aufsaugen mit 1,1 ml einer enteiweißten Serumprobe, die jeweils 10 µl einer definierten Glucosemenge enthielt, versetzt. Nach 10 Minuten wurde die Gesamtlösung durch Herabdrücken des Spritzenstempels in eine Küvette ausgedrückt. Bei einer Extinktion von 365 nm (F = 647) wurde die Glucosekonzentration in der Probe bestimmt. Verglichen mit den Ergebnissen, die bei dem üblichen Bestimmungsverfahren (manueller Test, analoge Testbedingungen) erhalten wurden, wurden folgende Glucosekonzentrationen [mg/100 ml] gefunden, die eine gute Übereinstimmung zeigen :

| Manueller Test | 50 | 80 | 100 | 165 | 200 | 370 | 435 | 500 |
|---|---|---|---|---|---|---|---|---|
| Matrixsystem | 50 | 79 | 102 | 161 | 204 | 365 | 427 | 485 |

Beispiel 3

Proteinbestimmung

Aus einem Polyurethan-Kaltschaum (Schaumstoff R 7295 der Firma Metzeler, Memmingen) wurden zylindrische Matrixsegmente (Ø 12 mm, Höhe 8 mm) ausgebohrt, ausgewaschen, getrocket und anschließend mit 0,5 ml einer Lösung getränkt, die

6

18 mmol/l K-Na-Tartrat,
10 mmol/l Kaliumjodid,
12 mmol/l Kupfersulfat und
200 mmol/l Natriumhydroxid

enthielt, und anschließend lyophilisiert. Die so hergestellten Reaktionsmatrices wurden in einer 5-ml-Einwegspritze je mit einer verschiedene Albuminmengen enthaltenden verdünnten Probelösung (10 µl Probe + 0,5 ml Wasser) versetzt. Durch Einführen und wiederholtes Herabdrücken eines mit Löchern versehenen Stempels wurde innerhalb von 10 Minuten die Lösung im Matrixsegment mehrmals in den Raum oberhalb der Matrix gepreßt und wieder in den Schaumstoff zurückgesaugt. Während dieser Zeit war der Auslauf der Spritze abgedichtet. Danach wurde der Stempel durch einen nicht durchlöcherten Stempel ausgetauscht, die Gesamtlösung in eine Küvette gedrückt und die Extinktion der Lösung bei 546 nm gegen den Blindwert der Reagenzlösung gemessen. Durch Umrechnung läßt sich die Proteinkonzentration der Ausgangsprobe bestimmen (F = 15) :

Protein [g/100 ml]

| eingesetzt | gefunden |
| --- | --- |
| 2,0 | 2,1 |
| 3,0 | 3,0 |
| 5,0 | 4,9 |
| 6,0 | 6,2 |
| 8,0 | 7,9 |
| 10,0 | 10,1 |
| 12,0 | 11,8 |

Beispiel 4

Bestimmung der Glutamat-Oxalacetat-Transaminase (GOT)

Aus einem Polyurethan-Polyesterschaum (Schaumstoff ME 6060 der Firma Metzeler, Memmingen) wurden zylindrische Matrixsegmente (Ø 20 mm, Höhe 30 mm) ausgebohrt und ausgewaschen. Die getrockneten Segmente wurden zur Hälfte (Segmente I) mit 3 ml einer Lösung getränkt, die
80 mmol/l Phosphatpuffer, pH 7,4,
200 mmol/l L-Aspartat,
0,18 mmol/l NADH (Nicotinamid-adenin-dinucleotid red.),
1 g/l Albumin,
$\geqslant$1,2 kU/l LDH (Laktatdehydrogenase) und
$\geqslant$0,6 kU/l MDH (Malatdehydrogenase)

enthielt und lyophilisiert. In entsprechender Weise wurde die zweite Hälfte der Segmente (Segmente II) mit 3 ml einer Lösung von

20 mmol/l Phosphatpuffer, pH 7,4,
25 mmol/l Oxoglutarat und
10 mmol/l Ethylendiamintetraacetat

versetzt und lyophilisiert. In einer 20-ml-Einwegspritze wurde jeweils ein Segment I mit einer durchlöcherten Polyethylenzwischenscheibe (Ø 20 mm, Dicke 2 mm) und einem Segment II überschichtet. Auf das Segment II wurden 2,5 ml einer verdünnten Standardserumprobe (500 µl Serum mit verschiedenen eingestellten GOT-Aktivitäten + 2 ml physiologische Kochsalzlösung) aufgegeben und nach 5 Minuten durch Herabdrücken des Spritzenstempels in das Segment I überführt. Dabei wurde nur soviel Druck angewendet, daß eine möglichst vollständige Überführung, nicht aber ein Verschieben der Zwischenscheibe bewirkt wurde. Nach weiteren 2 Minuten wurde durch vollständiges Herabdrücken des Stempels die Gesamtlösung in eine Küvette gepreßt und der Extinktionsverlauf bei 334 nm registriert. Aus der Steigung der erhaltenen Kurven läßt sich die Enzymkonzentration der Untersuchungsprobe berechnen (F = 1 471) :

7

## GOT [U/l]

| eingesetzt | gefunden |
|---|---|
| 2,9 | 3,2 |
| 5,3 | 5,0 |
| 14 | 14 |
| 23 | 21 |
| 40 | 38 |
| 57 | 58 |
| 74 | 76 |
| 109 | 111 |
| 143 | 140 |

## Beispiel 5

Enteiweißung und Glucosebestimmung

In einer 10-ml-Einwegspritze wurden getrennt durch eine mehrfach durchlöcherte Polyethylenscheibe (Ø 15 mm, Dicke 2 mm), ein Segment zur Glucosebestimmung (Beispiel 2) und ein Segment zur Enteiweißung (Beispiel 1) übereinander angeordnet. Zur Untersuchung des Glucosegehaltes von Seren wurden auf das obere mit Trichloressigsäure imprägnierte Segment jeweils 10 µl einer Serumprobe mit verschiedenen definierten Glucosemengen (Proteingehalt 5,5 mg/dl), wie in Beispiel 1 beschrieben, aufgegeben und mit 1,1 ml Wasser in das Segment zur Glucosebestimmung gepreßt. Dabei wurde nur soviel Druck angewendet, daß eine möglichst vollständige Überführung der Gesamtlösung, nicht aber ein Verschieben der Zwischenscheibe bewirkt wurde. Nach 10 Minuten wurde durch Durchdrücken des Stempels bis zum Anschlag die Gesamtlösung in eine Küvette gepreßt. Die Bestimmung der Glucosekonzentration erfolgte über eine Messung der Extinktion bei 365 nm. Man erhält analog Beispiel 2 einen linearen Meßbereich von 20 bis > 500 mg/dl Glucose.

## Beispiel 6

Glucosebestimmung

Aus einem Polyurethan-Polyesterschaum (Schaumstoff ME 6060 der Firma Metzeler Memmingen) wurden zylindrische Matrixsegmente (Ø 15 mm, Höhe 16 mm) ausgebohrt und ausgewaschen. Die getrockneten Segmente wurden mit 1 ml einer Lösung getränkt, die

20 mmol/l Phosphatpuffer, pH 7,5,
0,2 kU/l Diaphorase und
0,6 mol/l 3-(4-Jodphenyl)-2-(4-nitrophenyl)-5-phenyl-2H-tetrazoliumchlorid

enthielt und anschließend lyophilisiert. Die so hergestellten Reaktionsmatrices wurden in 10-ml-Einwegspritzen, getrennt durch eine mehrfach durchlöcherte Polyethylenscheibe, mit jeweils entsprechenden Segmenten Reaktionsmatrices zur Glucosebestimmung (vgl. Beispiel 2) überschichtet. Auf letztere wurden analog Beispiel 2 1,1 ml enteiweißte Serumprobe aufgegeben und die Gesamtlösung wurde nach 2-3 Minuten in das Diaphorase/Tetrazoliumsalz-Segment überführt. Nach 10 Minuten erfolgte die Auswertung über einen visuellen Farbvergleich der mit der Probe getränkten Matrix mit einer Farbvergleichstabelle bzw. über eine Extinktionsmessung (400-600 nm) der ausgepreßten Lösung. Es wurden analoge Resultate wie im Beispiel 2 erhalten.

## Beispiel 7

Enteiweißung und visuelle Glucosebestimmung

In einer 10-ml-Einwegspritze wurden — jeweils von mehrfach durchlöcherten Polyethylenscheiben

(Ø 15 mm, Dicke 2 mm) getrennt — von oben nach unten die Segmente zur Enteiweissung (Beispiel 1) sowie der Glucosebestimmung (Beispiel 6) übereinandergeschichtet. Auf die mit Trichloressigsäure beladene Matrix wurden jeweils 10 µl einer definierte Glucosemengen enthaltenden Serumprobe (5,5 mg/dl Proteingehalt) in der Mitte aufgetragen. Analog Beispiel 5 wurde die enteiweißte Probe anschließend zusammen mit der Spüllösung in den Glucosebestimmungsbereich und nach weiteren 2-3 Minuten in das Diaphorase/Tetrazoliumsalz-Segment überführt. Nach weiteren 10 Minuten erfolgte die Auswertung visuell mit Hilfe einer Farbvergleichstabelle bzw. über die Extinktion (578 nm) der ausgedrückten Lösung. Es wurden analoge Resultate wie in Beispiel 5 erhalten.

## Ansprüche

1. Testsystem zur Bestimmung von Inhaltsstoffen von Flüssigkeiten unter Verwendung preßfähiger, mit Reagenzien imprägnierter Matrices, dadurch gekennzeichnet, daß es mindestens eine imprägnierte, preßfähige Matrix in einem Reaktionsgefäß und eine Vorrichtung enthält, durch die die Matrix nach Aufnahme der zu untersuchenden Flüssigkeit unter Abgabe des Reaktionsproduktes komprimiert wird.

2. Testsystem nach Anspruch 1, dadurch gekennzeichnet, daß mehrere Matrices übereinander und durch flüssigkeitsdurchlässige Vorrichtungen voneinander getrennt angeordnet sind.

3. Testsystem nach den Ansprüchen 1 oder 2, dadurch gekennzeichnet, daß das Reaktionsgefäß die Gestalt einer Pipette oder einer Injektionsspritze aufweist.

4. Testsystem nach den Ansprüchen 1 bis 3, dadurch gekennzeichnet, daß die flüssigkeitsdurchlässigen Anordnungen Zwischenscheiben, Stege oder Verjüngungen des Reaktionsgefäßes sind.

5. Testsystem nach den Ansprüchen 1 bis 4, dadurch gekennzeichnet, daß die Matrices scheibenförmig gestaltet sind.

6. Testsystem nach den Ansprüchen 1 bis 5, dadurch gekennzeichnet, daß eine Matrix aus mehreren, mit verschiedenen Reagenzien imprägnierten Segmenten besteht.

7. Testsystem nach den Ansprüchen 1 bis 6, dadurch gekennzeichnet, daß zusätzliche Verteilungsschichten, vorzugsweise aus Filterpapier, über mindestens einer Matrix angebracht werden.

8. Testsystem nach den Ansprüchen 1 bis 7, dadurch gekennzeichnet, daß der Teil des Reaktionsgefäßes, der zur Aufnahme der aus der (letzten) Matrix austretenden Flüssigkeit dient, als Anzeigebereich gestaltet ist.

9. Testsystem nach den Ansprüchen 1 bis 7, dadurch gekennzeichnet, daß die Komprimierung der Matrices durch Stempel- oder Kolbendruck in einem zylindrischen Reaktionsgefaß erfolgt.

10. Verfahren zur Bestimmung von Inhaltsstoffen von Flüssigkeiten unter Verwendung preßfähiger, mit Reagenzien imprägnierter Matrices, dadurch gekennzeichnet, daß man die zu untersuchende Flüssigkeit auf eine imprägnierte, preßfähige Matrix aufbringt und das Reaktionsprodukt durch Komprimieren der Matrix aus dieser entfernt und entweder direkt oder erst nach einer oder mehreren weiteren Passagen durch imprägnierte Matrices einer Auswertung zuführt.

11. Verfahren nach Anspruch 10, dadurch gekennzeichnet, daß eine Passage nacheinander durch mehrere, verschiedenartig imprägnierte Matrices erfolgt, wobei jeweils das Reaktionsprodukt der einen Matrix in die folgende überführt wird.

12. Verfahren nach den Ansprüchen 10 und 11, dadurch gekennzeichnet, daß die Bestimmung durch Auswertung der im Reaktionsgefäß befindlichen Flüssigkeit erfolgt, die nach der Passage der (letzten) Matrix anfällt.

## Claims

1. A test system for estimating the constituents of liquids using compressible matrices impregnated with reagents, characterised in that it contains at least one impregnated compressible matrix in a reaction vessel and a device by means of which, after absorbing the liquid to be investigated, the matrix is compressed so as to release the reaction product.

2. A test system according to Claim 1, characterised in that several matrices are arranged above one another, separated from one another by devices which are permeable to liquids.

3. A test system according to Claims 1 or 2, characterised in that the reaction vessel has the shape of a pipette or an injection syringe.

4. A test system according to Claims 1 to 3, characterised in that the devices permeable to liquids are intermediate discs, spacing materials or constrictions of the reaction vessel.

5. A test system according to Claims 1 to 4, characterised in that the matrices have the shape of a disc.

6. A test system according to Claims 1 to 5, characterised in that a matrix consists of several segments impregnated with various reagents.

7. A test system according to Claims 1 to 6, characterised in that additional distribution layers, preferably consisting of filter paper, are mounted above at least one matrix.

8. A test system according to Claims 1 to 7, characterised in that the part of the reaction vessel which is used to receive the liquid emerging from the (last) matrix, has the form of an indicator zone.

9. A test system according to Claims 1 to 7, characterised in that the compression of the matrices is effected by the pressure of a plunger or cylinder in a cylindrical reaction vessel.

10. Method of estimating the constituents of liquids using compressible matrices impregnated with reagents, characterised in that the liquid to be investigated is applied to an impregnated compressible matrix and the reaction product is removed from the matrix by compressing the latter and is conveyed to evaluation either direct or only after one or more further passages through impregnated matrices.

11. Method according to Claim 10, characterised in that passage takes place successively through several matrices impregnated in a different manner, the reaction product of one matrix being transferred in each case into the following matrix.

12. Method according to Claims 10 and 11, characterised in that the estimation is effected by evaluating the liquid which is present in the reaction vessel and which is produced after passing through the (last) matrix.

**Revendications**

1. Système de test pour la détermination de constituants de liquides par utilisation de matrices compressibles imprégnées par des réactifs, caractérisé en ce qu'il contient au moins une matrice compressible imprégnée dans un récipient de réaction et un dispositif permettant de comprimer la matrice, après absorption du liquide à examiner, avec libération du produit de réaction.

2. Système de test selon la revendication 1, caractérisé en ce qu'il contient plusieurs matrices disposées les unes au-dessus des autres et séparées les unes des autres par des dispositifs perméables aux liquides.

3. Système de test selon les revendications 1 ou 2, caractérisé en ce que le récipient de réaction présente la forme d'une pipette ou d'une seringue pour injections.

4. Système de test selon les revendications 1 à 3, caractérisé en ce que les dispositifs perméables aux liquides sont des disques intermédiaires, des traverses ou des étranglements du récipient de réaction.

5. Système de test selon les revendications 1 à 4, caractérisé en ce que les matrices ont la forme de disques.

6. Système de test selon les revendications 1 à 5, caractérisé en ce qu'une matrice consiste en plusieurs segments imprégnés par des réactifs différents.

7. Système de test selon les revendications 1 à 6, caractérisé en ce qu'il contient, sur au moins une matrice, des couches de répartition supplémentaires, de préférence en papier-filtre.

8. Système de test selon les revendications 1 à 7, caractérisé en ce que la partie du récipient de réaction servant à recueillir le liquide sortant de la (dernière) matrice est conçue comme région indicatrice.

9. Système de test selon les revendications 1 à 7, caractérisé en ce que les matrices sont comprimées par la pression d'un piston ou d'un poinçon dans un récipient de réaction cylindrique.

10. Procédé pour doser les constituants de liquides par utilisation de matrices compressibles imprégnées par des réactifs, caractérisé en ce que l'on applique le liquide soumis à la mesure sur une matrice compressible imprégnée et on élimine le produit de réaction de la matrice en comprimant cette dernière puis on le soumet à la mesure, directement ou après un ou plusieurs autres passages au travers de matrices imprégnées.

11. Procédé selon la revendication 10, caractérisé en ce qu'il y a passage successif au travers de plusieurs matrices à imprégnations différentes, le produit de réaction de chaque matrice étant transféré dans chaque cas dans la matrice suivante.

12. Procédé selon les revendications 10 et 11, caractérisé en ce que la détermination est faite par mesure sur le liquide qui se trouve dans le récipient de réaction et qui a été obtenu après passage au travers de la (dernière) matrice.